Europäisches Patentamt

European Patent Office

Office européen des brevets

(11) Publication number: **0 193 309**
**B1**

(12) **EUROPEAN PATENT SPECIFICATION**

(45) Date of publication of patent specification: **23.05.90**

(21) Application number: **86300979.1**

(22) Date of filing: **13.02.86**

(51) Int. Cl.⁵: **A 61 F 13/15**

(54) **Paper diaper.**

(30) Priority: **14.02.85 JP 25177/85**

(43) Date of publication of application:
**03.09.86 Bulletin 86/36**

(45) Publication of the grant of the patent:
**23.05.90 Bulletin 90/21**

(84) Designated Contracting States:
**DE GB NL SE**

(56) References cited:
**US-A-3 965 906**
**US-A-4 282 874**
**US-A-4 321 924**

(73) Proprietor: **Nishino, Tetsuya**
**4-16-3-607 Kyonancho**
**Musashino-shi Tokyo (JP)**
(73) Proprietor: **Chiba, Yasuhiro**
**521-6 Fukasaku**
**Omiya City Saitama Prefecture 330 (JP)**

(72) Inventor: **Nishino, Tetsuya**
**4-16-3-607 Kyonancho**
**Musashino-shi Tokyo (JP)**

(74) Representative: **Funge, Harry et al**
**WILSON, GUNN & ELLIS 41-51 Royal Exchange**
**Cross Street**
**Manchester M2 7BD (GB)**

## Description

This invention relates to a paper diaper of which primarily element is fluff pulp, and particularly a paper diaper which is capable of diffusing and retaining fluid such as urine in a wide area both in longitudinal and lateral directions of the diaper, giving pleasant feeling in use.

Heretofore, many paper diapers utilize fluff pulp for absorbing the fluid. Referring to Fig. 7 which illustrates a typical structure of the known paper diaper, a fluff pulp layer "b" of a certain thickness is disposed on a suitable support paper "a", a vinyl sheet "c" covers underneath the support paper "a" and a nonwoven sheet "d" is placed on the fluff pulp layer "b" respectively, side edges "e" and "e'" are heat sealed, and both ends in the longitudinal direction, that is the direction perpendicular to the sheet of the drawing are also heat sealed to provide a finished product. As to specific shapes of the product, there are flat ones for the adult, folded ones to fit the groin to be fastened about the waist or rolled up ones with side edges shrinked for infants.

The fluff pulp which is used as a principal material of such paper diapers is produced relatively at a low cost and is well accepted by the industry. However, it is a mass of short fibers with the fibers little entwined to each other, so that it is unable to maintain a given shape and collapses easily. Since fibers are not entwined in the fluff pulp and there are formed substantial gaps between the fibers, there is generated no capillary action and the fluid cannot be transferred. Despite these shortcomings, the fluff pulp is generally used as a principal element of a liquid or fluid absorbing means.

Observing closely the condition of absorbing and retaining the excretion, particularly the fluid such as urine (to be referred to as the fluid hereinafter), there are several problems that must be settled. The discharged fluid passes through the external member, that is the nonwoven fabric "d" which is in direct contact with a human body to permeate and sink into the fluff pulp layer "b", but such permeation and sinking of the fluid is substantially confined or limited to a local area "R" enclosed by dotted lines in Fig. 7. This area "R" is coextensive with an area of the diaper where the fluid excreted from the human body makes contact with the diaper. Even if the fluid permeates and sinks into the thickness of the fluff pulp layer "b", there is no spreading of the fluid in the lateral direction "x" of the diaper. Likewise, there is no spreading of the fluid in the longitudinal direction. The fluid merely sinks down in substantially perpendicular direction "z" forcing the fluff pulp to cave-in. When a large quantity of fluid is discharged locally, the area "R" cannot retain the fluid within itself, and part of the fluid reaches on the surface of the vinyl sheet "c" at the bottom and starts flowing out therefrom. Some portion of the fluid seeps under the fluff pulp surrounding, and in contact with, the vinyl sheet, but since the fluff pulp is unable to absorb the fluid by itself, amount of the fluid that seeps is limited, with most of the fluid collecting on the vinyl sheet surrounding the area "R" and leaking laterally through side edges. In order to prevent this lateral leaking, it will become necessary to add some extra works, for example, to tighten that part of the diaper in contact with the groin into a cup like shape or to shrink the side edges. Such will merely increase the manufacturing cost while serving little purpose of dispersing or diffusing the fluid. Further, if the lateral leak is prevented by means of the work just described, the area "R" becomes a gorge of the diaper so to speak worn by a person. The fluid collects in the gorge like part in quantity wetting the area "R" drippingly while the fluff pulp outside the area "R" is not wet at all. This condition is felt by a wearer who feels discomfort in using the diaper. Thus, the fluff pulp which has been prepared for the purpose of absorbing and retaining the fluid turns out to be the cause of "fluid collection" bringing about a contrary effect by giving the thoroughly wet feeling. In addition, most of the remaining fluff pulp does not serve the purpose of absorbing and retaining the fluid which means a gross waste or uneconomical use of the material making up the diaper.

In order to reinforce the poor performance in absorbing and retaining the fluid as seen with the fluff pulp, it was proposed to use high absorbent polymer material together therewith. Referring to Fig. 7. the high absorbent polymer is inserted between the support sheet "a" and the fluff pulp layer "b". Pulverized polymer contained in the high absorbent polymer has a high degree of hydrophile property and is capable of absorbing the fluid 60 times its own weight, having the characteristics of taking in the fluid while it swells and is gelled. However, since it does not possess the property of dispersing the fluid, it cannot transfer the fluid to the remaining dry polymer even when the polymer under the area "R" is saturated with the fluid. It is not only incapable of solving the problem of local "fluid collection" of the fluff pulp but also presents another problem of increasing the product cost since the high absorbent polymer is expensive.

Another prior form of diaper is disclosed in U.S. Patent Specification Serial No. 4282874, such diaper comprising a highly moisture-absorbent fibrous pad interposed between a facing layer of a fibrous material and a moisture impermeable backing sheet, the pad including a perforated film provided between two cellulosic fibrous batts and the lowermost batt being of a greater density than the batt existing at the opposite side of the film and further including a highly compacted fibrous layer at its underside.

It is the object of the invention to provide a paper diaper which is pleasant to use yet inexpensive wherein the main element of diaper is the fluff pulp the whole of which is effectively utilized to the maximum to absorb and retain the fluid while an overall size of the diaper is minimized.

According to the present invention there is proposed a paper diaper including a moisture absorbent fibrous pad, and a surface layer of loosely compacted fluff pulp fibres at respective opposite sides of an essentially non-absorbent apertured or like partition layer, characterised by:

(a) a crust layer (5) arranged at the opposite side of the partition layer (3) with respect to the surface layer and in which the fluff pulp fibres are entwined and bonded to each other thereby to provide a means to diffuse the fluid falling down from the holes or the like of said partition layer (3) in an oriented manner and to retain the same; and

(b) a fluff pulp main layer (7) comprising fluff pulp fibres integrally continuous to a lower part and to both side edges of said crust layer (5).

In producing the diaper of the invention, a small amount of water is added to the surface of the fluff pulp main layer and pressure is applied to the surface in a given direction, except at the edge regions thereof, prior to heat drying. Thus in the outer surface layer, to a given thickness of fluff pulp and because fibers are not entwined to each other but merely form a mass which easily collapses, pulp fibers near the surface, aligned to a given direction, are bonded together. Thus a crust layer or ply having the strength not to collapse is formed continuously integral with a main body of fluff pulp underneath; the fluid is thereby caused to be dispersed into a wide area, to be retained therein by means of capillary action into a given direction (mainly in the longitudinal direction) created in the crust layer while non-water-absorbent or water resistant partition layer through which holes, grooves or the like are punched or formed (to be called partition layer) to allow the fluid to diffuse and fall down on the crust layer is provided on the crust layer integrally or separately, a thin fluff pulp surface layer or ply is placed on the partition layer to prevent rigid feeling or wet feeling caused by said partition layer transmitted to the user's skin.

In the paper diaper according to the invention, the fluff pulp of the surface layer or ply allows the fluid to permeate therethrough within a narrow area, the crust layer under the partition layer diffuses and retains the fluid over a wide area, so that the diaper of the invention conveys to the user a soft touch without giving wet feeling, and offers a pleasant feeling in use. The partition layer diffuses the fluid which passed through the fluff pulp of the surface layer or ply slightly wider than the local area where the fluid was discharged and permits the fluid to pass through holes or the like of the partition layer, so much so that the crust layer is allowed to exert, by means of capillary action, its inherent diffusion and retaining property, over much wider area to distribute and permeate the fluid in small quantity per unit area to the main layer or ply of fluff pulp at the forward and rear ends of the diaper which in the prior art goods remained substantially dry, thus causing the fluid to be dispersed and retained in a slightly moistened state inside substantial the whole area of the diaper. There is no collection of fluid at or in any of the partition layer, diffusion crust layer or the lower main layer or ply of fluff pulp so that, even the interior of the whole of the diaper gives the user moistened feeling, the diaper does not convey unpleasant dripplingly wet feeling, with the edges or ears of the main layer or ply of fluff pulp remaining at both sides of the crust layer effectively preventing lateral leaking of the fluid.

Embodiments of the invention will now be explained more in detail reference being made to the accompanying drawings in which:

Fig. 1 is a partially cross-sectioned view of the basic embodiment of the paper diaper of the invention showing the crossed face schematically,

Fig. 2 is a partial perspective view of the principal part of the paper diaper of Fig. 1,

Fig. 3 is a partial perspective view of another embodiment of the partition layer,

Fig. 4 is a partial perspective view of other embodiment of a separately formed partition layer,

Fig. 5 is a partial perspective view of a further embodiment of the partition layer,

Fig. 6 is a partial perspective view of yet further embodiment of separately formed partition layer, and Fig. 7 is a cross-sectional view of a known paper diaper. Figures 1 and 2 show basic embodiments of the invention, Fig. 1 showing schematically a partial cross-section of the paper diaper of the invention, and Fig. 2 is a partial enlarged perspective view of the principal part of the paper diaper of Fig. 1.

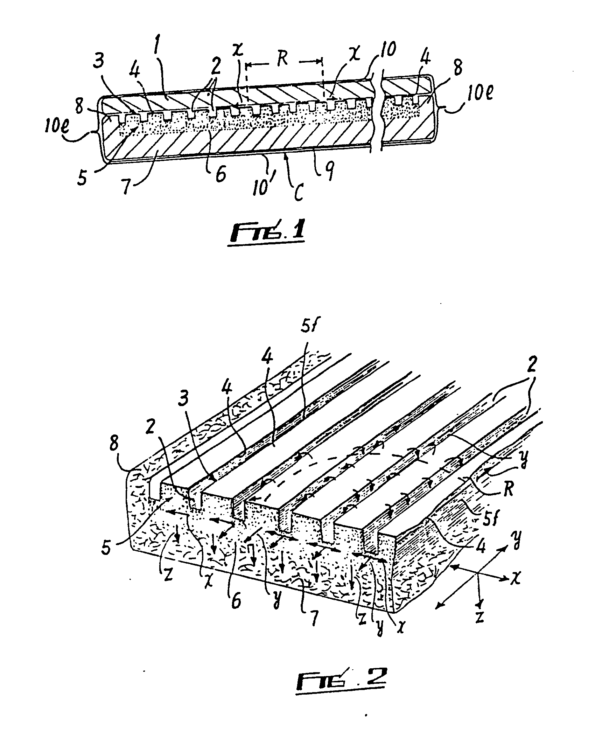

The paper diaper of the invention comprises mainly fluff pulp surface layer or ply 1 at the top, partition layer 3, with holes or the like, which is located under the surface layer which allows the fluid falling down from above to fall down without essentially absorbing it to a lower layer, crust layer 5 under the partition layer 3 which disperses the fluid that falls down in an orientated manner, namely in lateral direction x and predominantly in longitudinal direction y, and fluff pulp main layer or ply 7 inherently and integrally continuous to the crust layer 5, with the fluff pulp main layer 7 and the crust layer 5 continuous at the lower face 6 of the crust layer as well as at both sides. Side edges of the fluff pulp main layer 7 form ears 8. Under the fluff pulp main layer 7, there is placed water resistant paper 9. The whole of the above described components are wrapped in a nonwoven fabric. In Fig. 1, two upper and lower nonwoven fabrics 10 and 10' are set together as shown by reference 10e at side edges of the diaper and heat sealed.

Instead, it may be so arranged that one continuous sheet of nonwoven fabric encloses the whole diaper with the edges heat sealed at one side edge or at the center C. Although not shown in the drawing, both ends in the longitudinal direction are similarly heat sealed to present a completed paper diaper.

Fluff pulp is a mass of short fibers, and since short fibers are not essentially entwined with each other, it is a mass that is fragile and collapsible. Such untwined fluff pulp fibers are piled together and compressed or the like to form a surface layer or ply 1 of suitable thickness in which short fibers are arranged tightly. It is preferable to place suitable deodorant or aromatic under the surface layer 1 or to mix the same in the lower portion thereof. The deodorant or aromatic may be, according to the invention, mixed into fluff pulp in pulverized form or by spraying or the like when the fluff pulp fibers for the surface layer are caused to fall down from the loader to be piled.

In making the crust layer 5, a belt like body of fluff pulp of suitable thickness is placed on a belt, preferable air-permeable belt, air is sucked from below to tighten the piled fluff pulp, water in minute particles is applied to the surface of the belt like body of fluff pulp by spraying or splashing so that pulp fibers to a certain thickness from the surface absorb water. This applying of water should be carefully executed to avoid cave-in of fluff pulp or penetration through the thickness of the piled pulp sheet due to wetting of a localized area. Thickness of piled pulpsheet that does not absorb water makes fluff pulp main layer 7.

Next, pressure is applied to the surface of the fiber layer or ply that has absorbed water along substantially longitudinal direction of the belt like body of the fluff pulp in tangential direction to orient or align the fibers that absorbed water as much and predominantly as possible in the longitudinal direction while the oriented fibers are bonded tightly together leaving little gap between them.

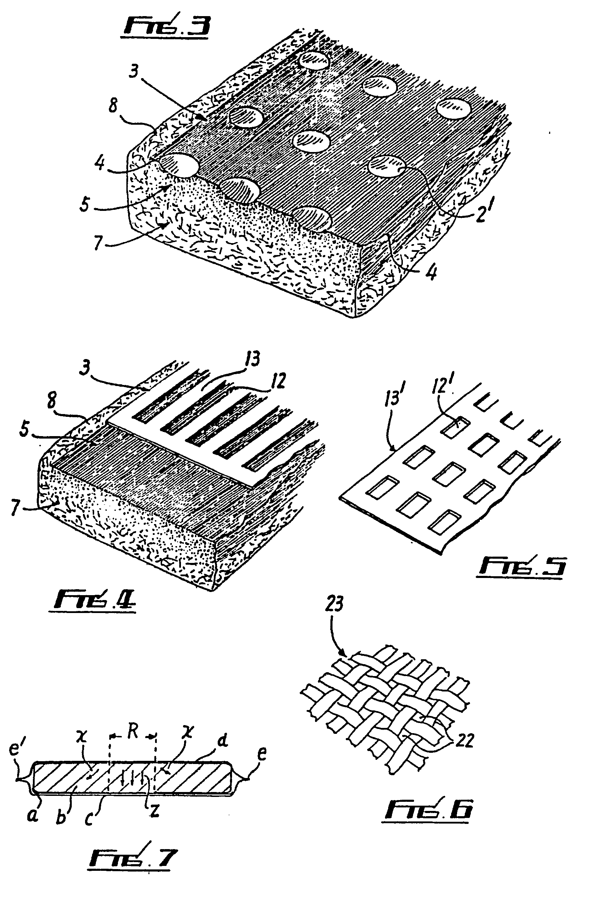

Then, by heat drying the fiber layer that absorbed water, the crust layer 5 is formed in which the fibers nearer to the surface are more closely bonded while maintaining their orientation. In the crust layer 5, the fibers, schematically shown in Fig. 2 as aligned fibers 5f, are caused to extend predominantly and substantially in the same direction, the fibers keeping minute gaps between each other, and thus there progresses a capillary action in that direction "y" while the fibers form an aggregated layer with sufficient strength with each fiber bonding to the other, so that, even when water seeps in, there will be no collapsing of the part that absorbed the water as seen with the mass of fluff pulp in which entwining of the fibers are not realized.

By adjusting the extent of application of minute water particle to the surface of fluff pulp as well as the degree of pressure applied, thickness of the abovedescribed crust layer and the degree of bonding and the orientation of fibers therein may be modified.

The lower part 6 of the crust layer 5 wherein the orientation and the degree of bonding are weak is inherently continuous to the fluff pulp main layer 7. In the main layer 7, fibers are not entwined nor oriented. In the drawing, the lower part 6 of the crust layer is shown by dotted line for the sake of convenience, but in reality there is not any clear and definite demarcation between the lower part 6 of the crust layer and the main layer 7. The crust layer 5 are also integrally continuous, at its both sides, to both side edges or ears 8 of the fluff pulp main layer 8.

In order to form partition layer 3, holes or gaps are formed to the surface of crust layer 5 made as described above. In the embodiments of Figures 1 and 2, there are formed a plurality of grooves 2 extending in the longitudinal direction. Grooves 2 may be formed by causing a roller having numbers of protrusions to make contact with the surface of the crust layer to make parts of the surface corresponding to said protrusions cave in. Thereafter, pharmaceutical such as water resistant agent or water repellant agent is applied in suitable manners to the remaining protruding surface of the crust layer to provide skin or film 4 which does not absorb water. In Fig. 2 the film 4 is shown as something that is not transparent, but, in reality, it may be made transparent so that fibers arranged in the crust layer 5 may be seen therethrough. Grooves 2 are drawn like square waves with sharp edges, but it will be understood that where grooves are made by pressing a roller having protrusions, edges and bottom of the groove take shapes that are not sharp. Thus, partition layer 3 is formed with a number of grooves 2 extending in longitudinal. direction with films 4 located between the grooves. Film 4 which does not absorb water is integral with the protruding surface of the crust layer 5, repels the fluid falling down thereon to let the fluid fall down further onto the groove 2, making the fluid to seep into the crust layer 5. The film 4 functions to prevent the fluid seeped in the crust layer 5 and fluff pulp main layer 7 to flow back to the surface layer 1 even when some pressure is applied thereto.

Fig. 3 is a partial perspective view of another embodiment showing other example of partition layer in which film 4 that does not absorb water is attached integrally with the crust layer 5 to which many dimples 2' are formed leaving the dimples 2' as they are. Film 4 is shown as a transparent element which allows the fibers of the crust layer to be seen therethrough.

Unlike the partition layer 3 integrally formed with the crust layer as explained above, it is possible to provide a partition layer separate from the crust layer. Fig. 4 shows such an example in a partial perspective view wherein partition layer 3 comprises water resistant sheet 13 made of paper, nonwoven fabric or the like to which water resistant or water repellant process is carried out and in which grooves 12 are formed. Water resistant sheet 13 is placed on the surface of crust layer 3.

Instead of grooves, water resistant sheet 13' having a number of windows 12' punched through it may replace partition layer 3 to be placed on the surface of crust layer 5. Window 12' may be a circular or oval hole.

As a separate partition layer, net 23 to which water resistant or water repellant process is carried out may be employed as shown in Fig. 6. The fluid passes through interstices 22, dispersed and falls down on the crust layer 5.

Now, fluid absorbing and retaining action of the paper diaper of the invention will be explained. As exemplified in Figures 1 and 2, discharged fluid passes through nonwoven fabric 10 and fluff pulp surface layer 1 within area "R" and reaches the patition layer 3. Since fluff pulp surface layer 1 is a mass of short fibers not entwined, the fluid does not stop in the surface layer 1, but soon reaches the punched partition layer 3 and falls into holes, gaps or grooves. Thus, fluff pulp surface layer 1 in contact with human body does not convey wet feeling, namely does not give unpleasant feeling. Further, the surface layer 1 serves as a buffer means to convey to the skin a feeling of soft touch.

On reaching film 4 of the non-absorbent partition layer 3 the fluid spreads in the longitudinal direction shown by arrows y in fig. 2 while some portion of the fluid spreads in the lateral direction shown by arrows x to fall into grooves 2, then to seep into the crust layer 5. Once the fluid seeps in the crust layer 5, the fluid is prevented from flowing backward by means of the partition layer 3. The crust layer 5 has capillary action predominantly in the longitudinal direction y, draws the fluid mainly in the longitudinal direction while drawing some portion in the lateral direction x, thus causing the fluid to be dispersed in both longitudinal and lateral directions, namely throughout the entire area of the paper diaper. The crust layer 5 does not only diffuses the fluid but also is capable to retain substantial amount of fluid by itself.

When the fluid that has been diffused and retained over wide area of the crust layer in the manner described above can no more be retained in the crust layer, the fluid seeps into fluff pulp main layer or ply 7 as shown by arrow z. Penetration of the fluid in the z direction does not take place within localized area R as explained in connection with the known article shown in Fig. 7, but takes place at many places over the wide area of the entire diaper, so that the amount of fluid that seeped is small per unit area. Because of this, the fluid is prevented from penetrating a certain local area of the fluff pulp layer 7 to collect at the bottom thereof, and the entire thickness of the fluff pulp main layer 7 both in longitudinal and lateral directions is evenly moistened by a very small amount of the fluid. Thus, the defect of the known diaper, wherein most of the main layer in the longitudinal direction except the local area R remained dry without absorbing the fluid is avoided, and the fluff pulp main layer 7 of the invention is effectively utilized. Water repellent paper 9 performs the primary function of supporting the main layer 7 which is slightly and evenly moistened all over as described above, and does not work like a saucer to collect the fluid penetrating through, and falling down from, the main layer 7. Therefore, that which supports the bottom of the water repellent paper 9 need not be a vinyl sheet as in the known art, but direct wrapping with a nonwoven fabric 10' which is gentle to the skin suffices. However, for the sake of safety, a vinyl sheet may be employed.

Ears 8 at both edges of the fluff pulp main layer 7 stop spreading out of the fluid in the lateral direction x from the punched partition layer 3 and diffusion crust layer 5. Since ears 8 are the mass of fibers not entwined, there are no diffusion and transfer of the fluid, so that the fluid does not leak out laterally. Ears 8 also serve as buffer when the diaper is applied to a body.

As has been described, the invention has the effect as follows: the discharge is widely diffused and distributed over the entire area of the diaper, so that maximum overall effective utilization of the main absorbent material is realized, without giving a dripplingly wet feeling to a user even when more fluid than before is absorbed, and if the diaper is to be changed when the same volume of discharge as before is excreted, the size of the diaper may be made smaller to improve the feeling when the diaper is strapped. Since edges of the fluff pulp which is the primary element of the diaper can completely prevent lateral leaking of the fluid, complex processing such as shrinking or rolling of the part of the diaper in contact with the groin may be omitted, and the use of expensive material such as high absorbent polymer is not necessary so that the manufacturing cost is reduced.

In summary, the paper diaper of the invention does not bring about fluid collection, and the fluff pulp main layer in contact with the skin is not noticeably wetted and gives a dry and soft feeling to the user which is effective in giving a pleasant feeling in use.

## Claims

1. A paper diaper including a moisture absorbent-fibrous pad, and a surface layer of loosely compacted fluff pulp fibres at respective opposite sides of an essentially non-absorbent apertured or like partition layer, characterised by:

(a) a crust layer (5) arranged at the opposite side of the partition layer (3) with respect to the surface layer and in which the fluff pulp fibres are entwined and bonded to each other thereby to provide a means to diffuse the fluid falling down from the holes or the like of said partition layer (3) in an oriented manner and to retain the same; and

(b) a fluff pulp main layer (7) comprising fluff pulp fibres integrally continuous to a lower part and to both side edges of said crust layer (5).

2. A paper diaper as claimed in claim 1, characterised in that said partition layer is formed with a plurality of grooves made on the top surface of said crust layer in longitudinal direction of the diaper.

3. A paper diaper as claimed in claim 1, characterised in that said partition layer comprises dents or dimples formed in the top surface of the crust layer and a non-absorbent film formed integrally with the top surface of the crust layer and apertured in register with the dents or dimples.

4. A paper diaper as claimed in claim 1, characterised in that said partition layer comprises a water resistant sheet or net, prepared separately from the crust layer, placed on the top surface of the crust layer.

5. A paper diaper as claimed in any one of the preceding claims, characterised by the presence of a deodorant or aromatic agent mixed in the lower part of the fluff pulp of the surface layer.

6. A paper diaper as claimed in any one of the preceding claims, further characterised in that the fluff pulp main layer includes ears arranged laterally of the crust layer.

## Patentansprüche

1. Papierwindel mit einer Flüssigkeit absorbierenden, faserigen Wattierung und einer Oberflächenschicht aus einem lose verdichteten Flaum aus Zellstoffasern an der einen bzw. der anderen Seite einer im wesentlichen nicht absorbierenden Trennschicht, die mit Öffnungen oder dergleichen versehen ist, gekennzeichnet durch:

a) eine Krustenschicht (5), die an der zu der Oberflächenschicht entgegengesetzten Seite der Trennschicht (3) angeordnet ist und in der die Fasern des Zellstoffflaumes verflochten und gegenseitig gebunden sind, um dadurch ein Mittel für das Ausbreiten von Flüssigkeit zu bilden, die durch die Öffnungen oder der gleichen der genannten Trennschicht (3) hindurch in ausgerichteter Weise nach unten gelangt, und um diese Flüssigkeit zurückzuhalten; und

b) eine Hauptschicht (7) aus Zellstofflaum, bei der die Fasern des Zellstofflaumes integral mit einem unteren Bereich und beiden Seitenrändern der genannten Krustenschicht (5) zusammenhängen.

2. Papierwindel nach Anspruch 1, dadurch gekennzeichnet, daß die genannte Trennschicht mit einer Mehrzahl von Nuten gebildet ist, die an der oberen Oberfläche der genannten Krustenschicht in Längsrichtung der Windel ausgebildet sind.

3. Papierwindel nach Anspruch 1, dadurch gekennzeichnet, daß die genannte Trennschicht Vorsprünge oder Vertiefungen aufweist, die in der oberen Oberfläche der Krustenschicht ausgebildet sind, und daß ein nicht absorbierender Film integral mit der oberen Oberfläche der Krustenschicht ausgebildet und mit auf die Vorsprünge oder Vertiefungen ausgerichteten Durchbrüchen versehen ist.

4. Papierwindel nach Ansprucn 1, dadurch gekennzeichnet, daß die genannte Trennschicht ein wasserbeständiges Blatt oder Netz aufweist, das getrennt von der Krustenschicht hergestellt und auf der oberen Oberfläche der Krustenschicht angeordnet ist.

5. Papierwindel nach irgendeinem der vorausgehenden Ansprüche, gekennzeichnet durch das Vorhandensein eines Deodorants oder eines aromatischen Mittels, das dem unteren Teil des Zellstofflaums der Oberflächenschicht beigemischt ist.

6. Papierwindel nach irgendeinem der vorausgehenden Ansprüche, dadurch gekennzeichnet, daß die Hauptschicht aus Zellstofflaum Ohren aufweist, die an der Krustenschicht seitlich angeordnet sind.

## Revendications

1. Couche en papier comprenant un coussinet fibreux absorbant l'humidité et une couche de surface en fibres de pulpe duveteuse tassées sans serrer sur des côtés opposés respectifs d'une couche de séparation à trous ou similaires, non absorbante, caractérisée par:

a) une couche de croûte (5) disposée, par rapport à la couche de surface, du côté opposé de la couche de séparation, et dans laquelle les fibres de pulpe duveteuse sont entrelacées et liées les unes aux autres pour fournir ainsi un moyen de diffusion orientée du fluide tombant des trous, ou similaires, de la dite couche de séparation (3) et retenir celui-ci, et

b) une couche principale de pulpe duveteuse (7) comprenant des fibres de pulpe duveteuse intégrées de manière continue à une partie inférieure et aux deux bords latéraux de la dite couche de croûte (5).

2. Couche en papier selon la revendication 1, caractérisée par le fait que ladite couche de séparation est formée avec une pluralité de rainures ménagées sur la surface de dessus de ladite couche de croûte en direction longitudinale de la couche.

3. Couche en papier selon la revendication 1, caractérisée par le fait que ladite couche de séparation comprend des dents ou ondulations formées dans la surface de dessus de la couche de croûte et un film non-absorbant formé intégré à la surface de dessus de la couche de croûte et muni de trous faisant face aux dents ou ondulations.

4. Couche en papier selon la revendication 1, caractérisée par le fait que ladite couche de séparation comprend une feuille, ou filet, préparée séparément de la couche de croûte, placée sur la surface de dessus de la couche de croûte.

5. Couche en papier selon l'une des revendications précédentes, caractérisée par la présence d'un agent désodorisant ou aromatique mélangé dans la partie inférieure de la pulpe duveteuse de la couche de surface.

6. Couche en papier selon l'une des revendications précédentes, caractérisée en outre par le fait que la couche principale en pulpe duveteuse comprend des oreilles disposées latéralement par rapport à la couche de croûte.

EP 0 193 309 B1

_FIG.1_

_FIG.2_

_FIG.3_

_FIG.4_

_FIG.5_

_FIG.6_

_FIG.7_